# FASCICULE DE BREVET EUROPEEN

(11) **EP 0 359 621 B1**
(45) Date de publication et mention de la délivrance du brevet: **28.07.1993**
(21) Numéro de dépôt: 89402391.0
(22) Date de dépôt: 01.09.1989
(51) Int. Cl.: C07C 63/49, A61K 49/02, C07B 59/00

(54) **Nouveau composé marqué au tritium, sa préparation et son application notamment dans la détermination de l'affinité des rétinoides pour leurs récepteurs nucléaires et leur protéine de liaison cytosolique**
Mit Tritium markierte Verbindung, ihre Herstellung und Verwendung, besonders bei der Bestimmung der Affinität der Retinoide für ihre nuklearen Rezeptoren und ihr zytosol-bindendes Protein
Compound labelled with tritium, its preparation and use, especially in the determination of the affinity of retinoids for their nuclear receptors and their cytosolic binding protein

(30) Priorité: 01.09.1988 FR 8811469
(43) Date de publication de la demande: 21.03.1990
(73) Titulaire: CENTRE INTERNATIONAL DE RECHERCHES DERMATOLOGIQUES GALDERMA - CIRD GALDERMA, F-06560 Valbonne (FR)
(72) Inventeur: Shroot, Braham, Chemin de Val Bosquet F-06600 Antibes (FR); Darmon, Yves-Michel, Vergers de Valconstance F-06600 Antibes (FR); Nedoncelle, Philippe, F-06130 Grasse (FR); Martin, Claude, Le Plan de Grasse F-06330 Grasse (FR)
(74) Mandataire: Tonnellier, Jean-Claude

(56) Documents cités:
- EP-A- 0 210 929
- DE-A- 3 142 975
- GB-A- 2 194 535

## Description

La présente invention a pour objet un nouveau composé marqué au tritium apparenté aux rétinoïdes, sa préparation et son application notamment dans la détermination de l'affinité des rétinoïdes pour leurs récepteurs nucléaires et leur protéine de liaison cytosolique et/ou dans la détermination de la teneur cellulaire en récepteurs nucléaires et en protéine de liaison cytosolique.

On sait que les rétinoïdes constituent une classe connue de composés qui agissent notamment sur la prolifération et la différenciation de nombreux types de cellules; voir par exemple B.A. Pawson et coll., Journal of Medicinal Chemistry, vol.25, n°11, pages 1269-1277 (1982).

Les rétinoïdes ont été utilisés en particulier dans le traitement de diverses maladies dermatologiques dans lesquelles un dérèglement des mécanismes de contrôle de la prolifération et de la différenciation des cellules épidermiques est impliqué; voir par exemple l'ouvrage "Update : Dermatology in General Medicine, edited by Thomas B.Fitzpatrick et al. (MacGraw-Hill Book Company), publié en 1983, en particulier le chapitre de D.B.Windhorst et al., The Retinoids, pages 226-237.

Le mode d'action des rétinoïdes est analogue à celui des stéroïdes et d'autres effecteurs interagissant avec des récepteurs nucléaires (Chytil & Ong, 1979). Des récepteurs nucléaires de l'acide rétinoïque (RARs) ont été isolés (Daly & Redfern, 1987) et les ADN complémentaires correspondants clonés et séquencés (Petkovich et al., 1987; Brand et al., 1988). Jusqu'à présent, trois récepteurs ont été décrits chez l'homme, RARa (462 résidus), RARb (448 résidus) et RAR γ (458 résidus). Il a d'autre part été montré qu'il existe dans le cytosol une protéine de liaison appelée CRABP (cellular retinoic acid binding protein), mais son rôle est mal connu.

La détermination de l'affinité d'un rétinoïde pour les RARs constitue un moyen particulièrement intéressant d'évaluation biologique potentielle dudit rétinoïde. La détermination de l'affinité pour la CRABP apporte un élément d'évaluation supplémentaire.

Plusieurs méthodes expérimentales permettent de déterminer l'affinité d'un ligand pour ses récepteurs ou protéines de liaison. On peut en particulier opérer par une méthode directe si le ligand considéré est marqué radioactivement, ou encore par compétition avec un ligand radioactif si le ligand considéré n'est pas marqué radioactivement.

Un bon ligand radioactif doit d'une part présenter une affinité élevée pour ses récepteurs et protéines de liaison et, d'autre part, présenter une faible fixation sur toute autre molécule. En outre, il doit être suffisamment stable pour permettre une utilisation commode.

Dans le cas des rétinoïdes acides, le ligand radioactif généralement utilisé est le plus souvent l'acide rétinoïque tritié, soit en position 2 (J.Labelled Compounds and Radiopharmaceuticals XVIII, p.1099 (1980)), soit en position 4 (demande de brevet allemand n°3.142.975).

L'acide rétinoïque présente certes une excellente affinité pour les RARs et la CRABP, avec une fixation non spécifique faible, mais la molécule présente l'inconvénient de se dégrader rapidement, par radiolyse, oxydation et photolyse.

Le document GB-A-2.194.535 décrit l'acide 2-(5,6,7,8-tétrahydro-5,5,8,8-tétraméthyl-2-naphtyl)-6-benzo[b]thiophènecarboxylique marqué au tritium, qui possède une activité spécifique d'environ 300 GBq/mole. Il est souhaitable d'obtenir des dérivés ayant une activité spécifique supérieure, notamment dans le but d'abaisser les seuils de détection dans les dosages.

On a maintenant découvert que l'acide 4-(5,6,7,8-tétrahydro-5,5,8,8-tétraméthyl-2-anthracenyl) benzoïque tritié constitue un nouveau ligand radioactif qui présente une excellente affinité pour les RARs et la CRABP, avec une fixation non spécifique faible. De plus, ce nouveau ligand est très stable et peut être obtenu avec une haute activité spécifique, ce qui le rend particulièrement utile pour la mesure de l'affinité des rétinoïdes pour les RARS et la CRABP.

La présente invention a donc pour objet l'acide 4-(5,6,7,8-tétrahydro-5,5,8,8-tétraméthyl-2-anthracenyl) benzoïque marqué au tritium.

L'invention a notamment pour objet un nouveau ligand radioactif, tel que défini ci-dessus, ayant une activité spécifique supérieure à 30 Ci/mmole et de préférence au moins égale à 50 Ci/mmole, soit environ 1875 GBq/mmole.

L'invention a également pour objet un procédé de préparation du nouveau ligand radioactif tel que défini ci-dessus. Ce procédé est principalement caractérisé par le fait que l'on prépare un alkyl ester multihalogéné de l'acide 4-(5,6,7,8-tétrahydro-5,5,8,8-tétraméthyl- 2-anthracenyl) benzoïque. En fonction du nombre d'équivalents de l'agent d'halogénation choisi, on peut introduire jusqu'à cinq atomes d'halogène. On soumet ensuite l'alkyl ester halogéné à l'action d'un agent réducteur tritié.

L'agent réducteur est par exemple du tritium utilisé en présence de palladium sur charbon à 10%, et de triéthylamine.

La réduction du dérivé halogéné est effectuée de préférence à température et sous pression ambiante, par exemple à 15-30°C sous une pression de 1 bar (soit environ 10⁵Pa).

L'alkyl ester est ensuite saponifié en milieu méthanolique, en présence de soude.

Le dérivé halogéné utilisé comme produit de départ est de préférence le dérivé bromé. L'alkyl ester utilisé est de préférence l'ester éthylique.

L'alkyl ester halogéné de l'acide 4-(5,6,7,8-tétrahydro-5,5,8,8-tétraméthyl-2-anthracenyl) benzoïque peut lui-même être préparé à partir de l'alkyl ester de l'acide 4-(5,6,7,8-tétrahydro-5,5,8,8-tétraméthyl-2-anthracenyl) benzoïque par l'action d'un agent d'halogénation (en particulier de bromation). Par exemple, le dérivé bromé peut être obtenu par l'action du brome dans l'acide acétique à température ambiante et sous atmosphère d'azote.

L'acide 4-(5,6,7,8-tétrahydro-5,5,8,8-tétraméthyl-2-anthracenyl) benzoïque est un produit connu (appelé ci-après composé I) et peut être obtenu selon le procédé décrit par exemple dans la demande de brevet européen n°86.401671 (210.929) déposée le 25 Juillet 1986.

Le ligand radioactif de l'invention peut également être obtenu au départ de l'alkyl ester de l'acide 4-(5,6,7,8-tétrahydro-5,5,8,8-tétraméthyl-2-anthracenyl) benzoïque par échange isotopique avec de l'eau tritiée en présence de platine et d'acide acétique à une température allant de préférence de 60 à 150°C. Dans ce cas l'échange isotopique se fait préférentiellement avec les protons aromatique du ligand.

L'invention a également pour objet l'utilisation de l'acide 4-(5,6,7,8-tétrahydro-5,5,8,8-tétraméthyl-2-anthracenyl) benzoïque tritié comme marqueur radioactif notamment dans la détermination de l'affinité, de la présence ou de la teneur des rétinoïdes et/ou dans la quantification des RARs et de la CRABP desdits rétinoïdes.

Par exemple, le marqueur radioactif de l'invention peut être utilisé lors de la purification des RARs et de la CRABP par les méthodes classiques de chromatographie d'exclusion sous basse ou haute pression : il suffit d'ajouter aux cellules (cas des RARs) ou à l'extrait tissulaire (cas de la CRABP) utilisés comme produits de départ, une quantité déterminée du ligand radioactif. Les RARs et la CRABP se trouvent ainsi marqués et on peut facilement repérer leur présence ou leur absence dans une fraction donnée.

La connaissance des courbes de fixation sur les RARs ou la CRABP permet également l'utilisation du marqueur de l'invention dans la quantification des RARs endogènes ou obtenus par des méthodes de transfection, ou dans la quantification de la CRABP.

L'invention a également pour objet l'utilisation du composé I tritié :
- comme marqueur radioactif pour repérer, doser ou marquer les récepteurs nucléaires de l'acide rétinoïque ;
- comme marqueur radioactif pour mesurer l'affinité des rétinoïdes pour les RARs (expériences de compétition) et avoir ainsi une estimation de leur activité biologique ;
- comme marqueur radioactif pour repérer, doser ou marquer la CRABP ; ou pour doser un rétinoïde connu par compétition pour la fixation sur la CRABP ;
- comme marqueur radioactif pour mesurer l'affinité des rétinoïdes pour la CRABP.

Le marqueur radioactif de l'invention peut également être utilisé dans la caractérisation d'anticorps contre le composé I, ces anticorps (obtenus selon les méthodes classiques d'obtention d'anticorps anti-haptènes) étant eux-mêmes utilisables dans la détermination de la quantité du produit I fixé ou non sur les RARs et/ou la CRABP. Les anticorps marqué sont utilisés pour réaliser un radioimmunodosage.

Le marqueur radioactif de l'invention peut aussi être utilisé dans l'étude du mécanisme d'action intracellulaire et du métabolisme général du composé I in vivo et dans les cellules et extraits cellulaires, et aussi pour étudier la distribution in vivo ainsi que cellulaire et subcellulaire de ce composé et des rétinoïdes en général.

L'exemple suivant illustre l'invention sans toutefois la limiter.

### EXEMPLE 1

### Acide 4-(5,6,7,8-tétrahydro-5,5,8,8-tétraméthyl-2-anthracenyl) benzoïque marqué au tritium :

a) L'ester éthylique correspondant à l'acide cité (250 mg; 0,644 mmole) est mis en solution dans 25ml d'acide acétique sous atmosphère d'azote. On ajoute 1ml d'une solution de brome dans l'acide acétique (230 mg de brome par ml, soit 2,2 équivalents).
   Le mélange est agité 4 heures à température ambiante, versé sur 100ml d'eau, puis extrait au dichlorométhane (100ml).
   La phase organique est séparée, lavée à l'eau puis à l'aide d'une solution saturée d'hydrogènocarbonate de sodium et enfin de thiosulfate de sodium.
   On sèche sur sulfate de magnésium anhydre, puis le solvant est évaporé sous pression réduite.
   On obtient 350mg d'un solide jaune clair correspondant à un produit bromé d'après son spectre de masse.
b) Le composé bromé obtenu est ensuite tritié. 19,3mg de précurseur bromé sont solubilisés dans un mélange composé de 2ml d'éthanol et 1ml de THF.
   On ajoute 17µl de triéthylamine, et 14mg de palladium sur charbon à 10%.
   Le milieu réactionnel est agité pendant 2 heures sous atmosphère de tritium (1 atmosphère), puis le catalyseur est filtré, les labiles éliminés par 2 fois 10ml d'un mélange composé de 50% d'éthanol et 50% d'acétonitrile.
   Le produit est repris par 100ml d'éthanol.
   On obtient 1.500 mCi d'ester éthylique tritié.
c) Le produit tritié précédent (1,5 Ci) est solubilisé dans 5ml de méthanol, 1ml de soude 5N est ajouté et le mélange résultant est chauffé à reflux pendant 2 heures, refroidi, acidifié à l'aide de 2ml d'acide chlorhydrique 6N, puis extrait par 50ml d'éther éthylique.
   La phase éthérée est décantée, lavée à l'eau, séchée sur sulfate de magnésium anhydre, et les solvants évaporés sous pression réduite. Le résidu obtenu est dissous dans le minimum de dichlorométhane et purifié par chromatographie liquide à haute performance (éluant : dichlorométhane/mé- thanol 9:1).
   Le produit final est dissous dans 100ml de méthanol. Sa pureté est vérifiée par chromatographie sur couche mince (Silice; éluant comme ci-dessus). Une seule tache est apparente sous irradiation UV (254 et 366 nm) et lors du comptage bêta.
   Par HPLC (phase inverse, colonne ZORBAX ODS, éluant : acétonitrile/eau/acide trifluoracétique 95:4,9:0,1), un seul pic est détecté par UV (254 nm) et par détection bêta (scintillation liquide en continu).
   L'activité spécifique déterminée par spectrophotométrie UV, puis comptage bêta, est de 52,8 Ci/mmole.
   L'activité totale obtenue, mesurée par comptage au scintillateur liquide, est de 1,35 Ci.

### Caractéristiques de la fixation du composé de l'exemple 1 sur les récepteurs nucléaires RARs

Des cultures de cellules F9 de carcinome embryonnaire murin (habituellement 4 boîtes de 90 mm contenant au total 2-4x10⁷ cellules par condition d'incubation) sont lavées une fois avec du PBS (tampon phosphate salin) et incubées pendant 3 heures en milieu sans sérum supplémenté avec le rétinoïde marqué en absence ou en présence de rétinoïde froid homologue ou hétérologue (pour vérifier la spécificité et pour les expériences de compétition). Les cellules sont alors détachées avec un mélange trypsine-EDTA et numérées avec un hémocytomètre de Burker. Le nucléosol contenant des RARs est extrait à partir des noyaux purifiés selon la méthode décrite par Daly et Redfern (1987), (c'est-à-dire après traitement par l'ADNase et le NaCl 0,6M) et analysé par chromatographie d'exclusion de haute performance (HPSEC). 50µl d'extrait marqué sont injectés sur une colonne GF250 (Dupont de Nemours) de 9x250 mm. L'élution est réalisée dans un tampon 0,3M KH₂PO₄ pH 7,8 à un débit de 1ml/min. Le contenu en protéine est suivi par mesure de densité optique à 280nm. 28 fractions de 0.3 ml chacune sont collectées et comptées dans du scintillant Picofluor (Packard). Pour chaque concentration de rétinoïde le nombre de molécules liées est déterminé à partir de la surface du pic de radioactivité et la concentration de demi-saturation (C50) calculée. Dans les expériences de compétition on calcule les concentrations inhibitrices 50% (IC50), le composé radioactif de l'exemple I étant utilisé à une concentration de 2.10⁻⁹M. La calibration de poids moléculaire de la colonne est effectuée à l'aide d'albumine humaine (67kDa), d'ovalbumine (45kDa) et de myoglobine (16,8kDa). Les courbes dose-réponse et les courbes de compétition sont analysées de manière informatique par régression non-linéaire en utilisant les diverses formes de l'équation de Clark.

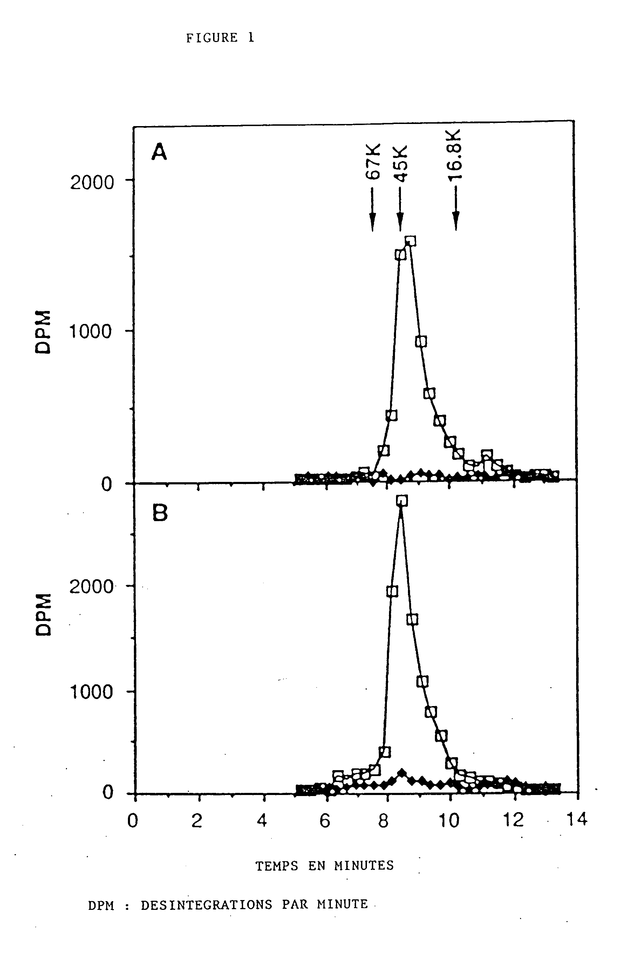

La figure 1 représente le profil d'HPSEC dans la détection des récepteurs nucléaires de l'acide rétinoïque (RARs) dans une fraction d'approximativement 45kDa dans le nucléosol des cellules F₉, et permet d'étudier la spécificité de la fixation.

Les cellules F₉ ont été incubées avec 200nM d'acide rétinoïque tritié (A) ou du composé de l'exemple 1 tritié (B) en absence ( □ - □ ) ou présence (♢ - ♢) de 20.000 nM de ligand homologue froid.

Quand la méthode décrite ci-dessus est utilisée et que les cellules F9 sont incubées avec le composé de l'exemple 1 à une concentration de 200nM, un pic unique de radioactivité est observé dans l'extrait nucléaire (Figure 1B). Le poids moléculaire correspondant à la fraction moyenne du pic est de 45kDa approximativement, un chiffre proche du poids moléculaire des RARs humains, ainsi qu'il est déduit de la séquence de leurs ADNc (Petkovich et al., 1987; Brand et al., 1988), et compatible avec le coefficient de sédimentation 4S des récepteurs murins (Daly & Redfern, 1987). La fixation est spécifique puisqu'elle est abolie quand les cellules sont coincubées avec un excès de 100 fois du composé I froid (Figure 1B). On ne trouve pas de CRABP dans l'extrait nucléosolique de F9, comme déjà souligné par Daly et Redfern (1987).

D'autre part, par transfection de lignées cellulaires ne contenant pas de récepteurs des rétinoïdes avec des vecteurs d'expression codant isolément pour chaque récepteur des rétinoïdes, il est possible d'isoler des extraits cellulaires contenant chaque récepteur et d'effectuer un test de liaison selon le protocole décrit ci-dessus. On peut ainsi déterminer l'affinité du composé de l'exemple I pour chaque récepteur des rétinoïdes.

### Comparaison avec la fixation de l'acide rétinoïque tritié sur les récepteurs nucléaires RARs

La figure 1A montre le profil d'HPSEC obtenu après traitement des cellules avec 200nM d'acide rétinoïque tritié (activité spécifique = 52,5 Ci/mmole). On peut noter que le pic radioactif élue à la même position qu'avec le composé de l'exemple 1. D'autre part, le pic n'est pas observé quand les cellules ont été coincubées avec 20000nM d'acide rétinoïque froid.

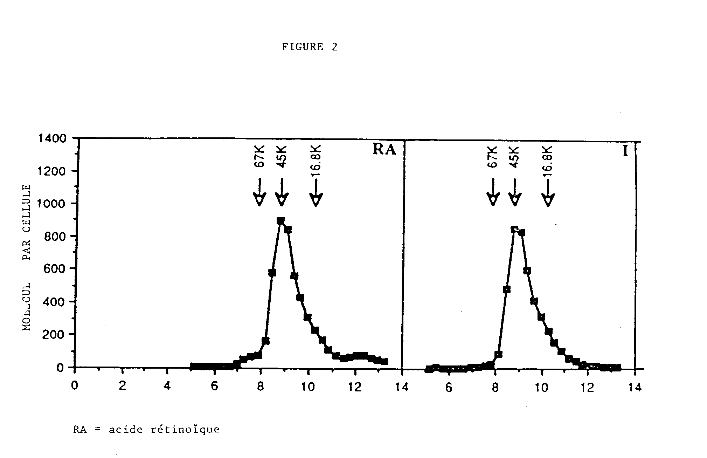

La figure 2 donne les résultats concernant la fixation de l'acide rétinoïque et du composé de l'exemple 1 sur les RARs. Les deux composés ont été utilisés à une concentration de 200 nM. La quantité de ligand liée aux RARs est exprimée en molécules par cellule.

Si on normalise les résultats d'HPSEC en fonction du nombre de cellules, c'est-à-dire en les exprimant par molécules de ligand fixés aux RARs par cellule, le composé de l'exemple 1 et l'acide rétinoïque donnent un pic de même surface correspondant à approximativement 4.000 molécules par cellule (Fig. 2).

Pour déterminer la concentration du composé de l'exemple 1 donnant 50% de saturation des RARs (C50), une courbe de saturation a été tracée en exprimant la surface du pic radioactif en fonction de concentrations variées du composé de l'exemple 1. La C50 est comprise entre 0,5 et 2 nM.

### Détermination de l'affinité des rétinoïdes pour les RARs par compétition avec le composé de l'exemple 1 tritié

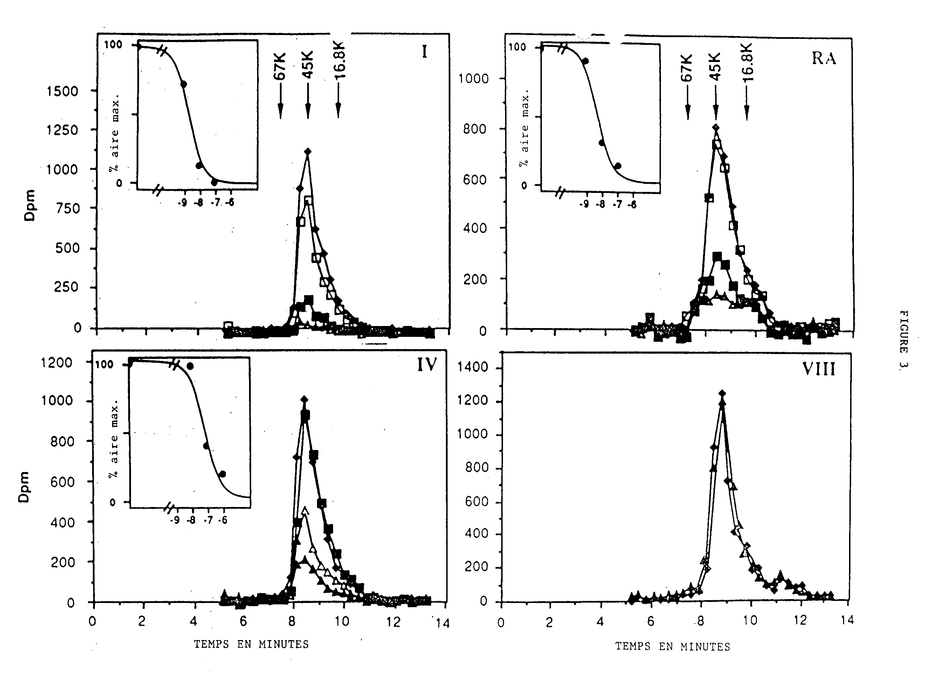

Compte-tenu de sa stabilité et de son excellente affinité pour les RARs, le composé de l'exemple 1 permet en principe de déterminer par compétition l'affinité de n'importe quel rétinoïde pour les RARs. La figures 3 montrent des expériences de compétition réalisées avec une concentration fixe de 2 nM du composé de l'exemple 1 et des concentrations variables d'autres rétinoïdes (10⁻⁶M à 10⁻¹⁰M). Les exemples de rétinoïdes cités n'ont pas un caractère limitatif.

Les profils d'HPSEC sont obtenus après incubation des cellules F9 avec 2x10⁻⁹M du composé de l'exemple 1 en absence (♢ - ♢) ou en présence de 10⁻⁹M (□ - □), 10⁻⁸M ( ■ - ■ ), 10⁻⁷M ( △ - △ ), 10⁻⁶M (▲ - ▲ ) de compétiteurs froids. I = composé de l'exemple 1 non marqué. RA=acide rétinoïque. Les sigmoïdes d'inhibition rapportées en encarts ont permis de déterminer les IC50 rapportées dans la table 1. Pour chaque concentration de compétiteur, la surface du pic est exprimée en pourcentage de la surface obtenue avec le composé de l'exemple 1 sans compétiteur.

En mesurant la surface du pic radioactif pour chaque concentration de compétiteur froid, il est possible de tracer des sigmoïdes de compétition. Un traitement informatique de ces courbes permet de déterminer l'IC50 (concentration inhibitrice 50%) de chaque rétinoïde. A titre de contrôle, on a aussi mesuré l'IC50 du composé I froid, et on a aussi utilisé un rétinoïde inactif biologiquement (composé VIII). Le Tableau 1 montre les valeurs d'IC50 obtenues. On remarque que le composé I a une affinité pour les RARs 6 fois meilleure que celle de l'acide rétinoïque lui-même.

### Corrélation entre l'activité biologique des rétinoïdes et leur affinité pour les RARs telle qu'elle est déterminée par compétition à l'aide du composé de l'exemple 1

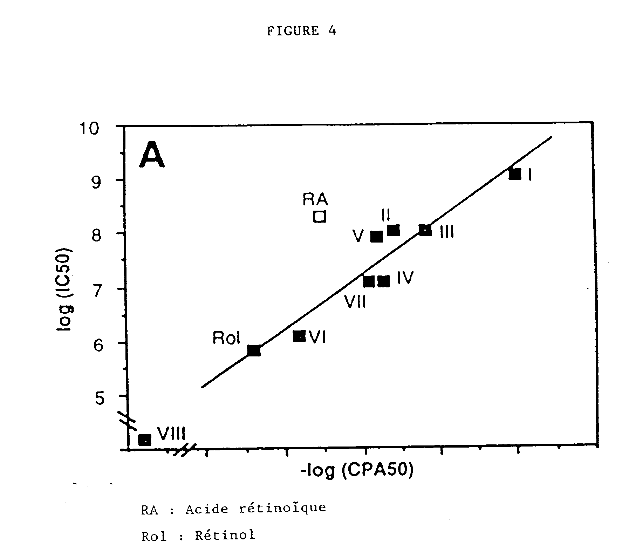

L'activité biologique des rétinoïdes peut être mesurée sur les cellules F9 en quantifiant la production d'activateur du plasminogène; sécrété consécutivement à la différenciation de ces cellules, provoquée par les rétinoïdes. La CPA50 est la concentration de rétinoïdes provoquant une induction de 50% du maximum d'activateur du plasminogène sécrété. En compararant les valeurs d'IC50 et de CPA50 du Tableau 1, on peut remarquer qu'il existe une excellente corrélation entre l'affinité des rétinoïdes pour les RARs et leur activité biologique (voir aussi la droite de régression de la Figure 4). La seule exception est l'acide rétinoïque lui-même. Ce fait s'explique par la grande instabilité de ce produit. En effet, la mesure d'IC50 s'effectue après incubation de 3 heures tandis que la mesure de CPA50 s'effectue après incubation de 3 jours.

Sur la Figure 4 on a représenté la régression linéaire montrant une excellente corrélation entre l'activité biologique des rétinoïdes (CPA50) et leur affinité pour les RARs. Les valeurs correspondant à l'acide rétinoïque (RA = □ ) n'ont pas été utilisées. L'affinité pour les RARs et l'activité biologique du composé VIII sont en dessous du seuil de détection.

### Caractéristiques de la fixation du composé de l'exemple 1 sur la protéine de liaison CRABP

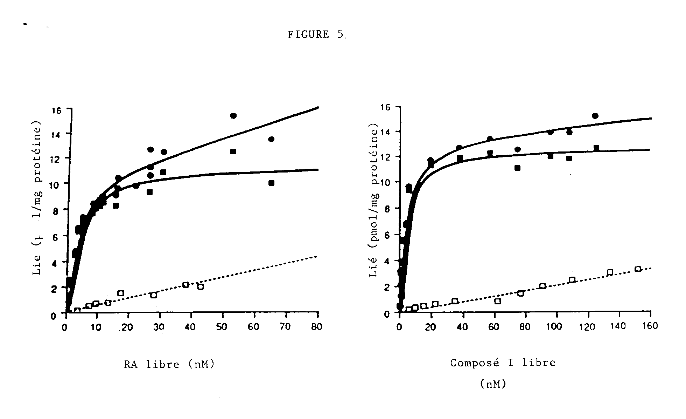

Les résultats concernant la fixation du composé I et de l'acide rétinoïque sur la CRABP sont représentés à la Figure 5. On étudie la fixation directe de concentrations croissantes du composé de l'exemple 1 (b) et d'acide rétinoïque (a) à du cytosol de testicule de rat contenant la CRABP en absence (● - ●) ou en présence de 1000 nM de ligand froid homologue. La différence ( ■ - ■ ) représente la liaison spécifique.

La fixation du composé revendiqué sur la CRABP a été caractérisée par des expériences de saturation (voir Figure 5b). La fixation totale (non spécifique + spécifique (1 - 1) est obtenue par incubation de concen- trations croissantes (jusqu'à un maximum de 125 nM) du composé revendiqué avec une quantité constante (0,3 ml) d'un homogénat de testicules de rat (organe riche en CRABP). La fixation non spécifique du composé tritié est déterminée en parallèle, par la mesure de la fixation avec des concentrations croissantes du ligand radioactif en présence d'un large excès (1 µM) de ligand froid homologue ( □ - □ ). Un temps d'incubation de deux heures est nécessaire à l'établissement de conditions d'équilibre.

Une fois l'équilibre atteint, le complexe CRABP-ligand tritié est séparé du ligand non lié par fltration sur colonne de gel d'exclusion Sephadex G25 (colonnes BIO-Rad econo, 0,5 x 20 cm) : le complexe est élué (pic d'élution à 2,5 ml), tandis que le ligand non lié est retenu en totalité sur la colonne. Il en résulte une séparation totale entre produit lié et non lié. L'élution est faite directement dans des tubes à scintillation et la radioactivité est mesurée par comptage.

Les résultats ainsi obtenus sont analysés par régression non-linéaire utilisant comme modèle l'équation de Clark (A.J. Clark, The mode of action of drugs on cells; A. Arnold and Co., London, 1933).

Sur la figure 1, on a représente en abscisses les concentrations initiales du ligand étudié, et en ordonnées, les concentrations à l'équilibre du complexe ligand-protéine de liaison.

La différence entre la courbe de fixation totale ( ● - ● ) et de fixation non spécifique ( □ - □ ) donne la courbe de fixation spécifique ( ■ - ■ ). L'analyse de cette courbe permet d'obtenir la constante de dissociation à l'équilibre (Kd) qui est la concentration de produit nécessaire pour saturer 50% de la CRABP. Cette constante est une mesure de l'affinité du ligand pour sa protéine de liaison, le Kd étant inversement proportionnel à l'affinité (plus le Kd est faible, plus l'affinité est grande).

Cette analyse donne une valeur de Kd de 4 nM. La fixation spécifique est saturable et réversible. La fixation non spécifique est linéaire et non saturable dans la gamme de concentrations utilisées, et représente moins de 10% de la fixation totale. La concentration de CRABP dans le cytosol de testicule de rat utilisé pour l'expérience peut être déterminée dans les expériences de liaison où les rétinoïdes marqués sont utilisés à concentration saturante. On trouve une valeur de 10-12 pmol/mg protéine aussi bien avec le composé revendiqué qu'avec l'acide rétinoïque. D'autre part, on peut déterminer par HPSEC que ces deux ligands se fixent sur une molécule d'un poids moléculaire de 15 kDa (poids moléculaire de la CRABP) avec le même profil d'élution.

### Comparaison avec l'acide rétinoïque tritié

Dans les mêmes conditions, on peut étudier la fixation de l'acide rétinoïque, sur la CRABP (Figure 5a). La valeur du Kd de l'acide rétinoïque est 2 nM, la fixation non spécifique représentant approximativement 25% de la fixation totale.

### Détermination de l'affinité d'un rétinoïque pour la CRABP par compétition avec le composé revendiqué marqué

Ces expériences de compétition sont effectuées de la façon suivante : on mélange une quantité fixe de ligand radioactif (correspondant sensiblement à la demi-saturation, dans notre cas 4 nM), avec des quantités croissantes de ligand froid (non radioactif). Puis on ajoute la protéine de liaison et on détermine la quantité de ligand radioactif fixée à l'équilibre (en % de la fixation initiale en l'absence de ligand froid, pour la quantité de CRABP considérée).

A partir des courbes de compétition, on détermine dans chaque cas la IC50 (concentration de ligand froid réduisant l'attachement du ligand tritié de 50%). Cette valeur IC50 permet de calculer dans chaque cas la constante de dissociation à l'équilibre (Kd) pour le ligand froid.

Le Tableau 1 indique les valeurs qui ont été déterminées pour les différents composés étudiés. Des valeurs identiques ont été déterminées si au lieu du composé revendiqué, l'acide rétinoïque lui-même marqué a été utilisé dans les expériences de compétition.

**TABLEAU 1**

| Comparaison de l'activité biologique des rétinoïdes et de leur affinité pour les RARs et la CRABP | | | |
|---|---|---|---|
| | ACTIVITE BIOLOGIQUE CPA50 (nM) | AFFINITE | |
| | | RARs:IC50(nM) | CRABP:Kd(nM) |
| Acide rétinoïque | 350 | 5 | 2 |
| Rétinol | 2.500 | 1.400 | 600 |
| Composé I | 1 | 0,8 | 4 |
| Composé IV | 53 | 77 | 2 |
| Composé V | 66 | 12 | 20 |
| Composé VI | 670 | 770 | 200 |
| Composé VII | 85 | 76 | 200 |
| Composé VIII | >10.000 | >10.000 | 200 |
| NB : Composé I = composé de l'exemple 1 froid (non marqué) Composé IV = Acide 2-(5,6,7,8-tétrahydro-5,5,8,8-tétraméthyl-2-naphtyl)-6 benzo(b)thiophène carboxylique Composé V = Acide 6-(5,6,7,8-tétrahydro-5,5,8,8-tétraméthyl-2-naphtyl-2-naphtoïque Composé VI = Acide 2-(5,6,7,8-tétrahydro-5,5,8,8-tétraméthyl-2-naphtyl)-6 benzo(b)furan carboxylique Composé VII = Acide 4-(5,6,7,8-tétrahydro-5,5,8,8-tétraméthyl-2-naphtamido) benzoïque Composé VIII = Acide 1-méthyl-4-thiol-6-(5,6,7,8-tétrahydro-5,5,8,8-tétraméthyl-2-naphtyl-2-naphtoïque | | | |

## Revendications

1. Acide 4-(5,6,7,8-tétrahydro-5,5,8,8-tétraméthyl-2-anthracényl) benzoïque marqué au tritium.

2. Acide 4-(5,6,7,8-tétrahydro-5,5,8,8-tétraméthyl-2-anthracényl) benzoïque tritié, tel que défini dans la revendication précédente, caractérisé par le fait qu'il possède une activité spécifique d'au moins 1875 GBq/mmole.

3. Procédé de préparation d'un produit tritié tel que défini dans l'une quelconque des revendications précédentes, caractérisé par le fait que:
- soit l'on prépare un alkyl ester multihalogéné d'acide 4-(5,6,7,8-tétrahydro-5,5,8,8-tétraméthyl-2-anthracényl) benzoïque et le soumet à l'action d'un agent réducteur tritié ou du tritum en présence d'un catalyseur puis à une saponification;
- soit l'on soumet l'alkyl ester d'acide 4-(5,6,7,8-tétrahydro-5,5,8,8-tétraméthyl-2-anthracényl) benzoïque à une réaction d'échange isotopique avec de l'eau tritiée en présence de platine et d'acide acétique à une température de 60° à 150°C.

4. Procédé selon la revendication 3, caractérisé par le fait que l'agent réducteur est le tritium.

5. Procédé selon selon l'une quelconque des revendications 3 et 4, caractérisé par le fait que le produit halogéné est obtenu par l'action d'un agent d'halogénation sur un alkyl ester de l'acide 4-(5,6,7,8-tétrahydro-5,5,8,8-tétraméthyl-2-anthracényl) benzoïque.

6. Procédé selon l'une quelconque des revendications 3 à 5, caractérisé par le fait que l'agent d'halogénation est un agent de bromation.

7. Procédé selon l'une quelconque des revendications 3 à 5, caractérisé par le fait que l'alkyl ester utilisé est l'ester éthylique.

8. Utilisation de l'acide 4-(5,6,7,8-tétrahydro-5,5,8,8-tétraméthyl-2-anthracényl) benzoïque tritié comme marqueur radioactif pour repérer, doser ou marquer les récepteurs nucléaires de l'acide rétinoïque, endogènes ou obtenus par des méthodes de transfection.

9. Utilisation de l'acide 4-(5,6,7,8-tétrahydro-5,5,8,8-tétraméthyl-2-anthracényl) benzoïque tritié comme marqueur radioactif pour mesurer l'affinité des rétinoïdes pour les RARs (expériences de compétition) et avoir une estimation de leur activité biologique.

10. Utilisation de l'acide 4-(5,6,7,8-tétrahydro-5,5,8,8-tétraméthyl-2-anthracényl) benzoïque tritié comme marqueur radioactif pour repérer, doser ou marquer la CRABP.

11. Utilisation de l'acide 4-(5,6,7,8-tétrahydro-5,5,8,8-tétraméthyl-2-anthracényl) benzoïque tritié comme marqueur radioactif pour mesurer l'affinité des rétinoïdes pour la CRABP.

12. Utilisation de l'acide 4-(5,6,7,8-tétrahydro-5,5,8,8-tétraméthyl-2-anthracényl) benzoïque tritié comme marqueur radioactif pour doser un rétinoïde connu par compétition pour la fixation sur la CRABP.

13. Utilisation de l'acide 4-(5,6,7,8-tétrahydro-5,5,8,8-tétraméthyl-2-anthracényl) benzoïque tritié comme marqueur radioactif pour la caractérisation d'anticorps dirigés contre ce composé et l'utilisation conjointe du composé et des anticorps pour réaliser un radioimmunodosage.

14. Utilisation de l'acide 4-(5,6,7,8-tétrahydro-5,5,8,8-tétraméthyl-2-anthracényl) benzoïque tritié comme marqueur radioactif pour étudier la distribution in vivo ainsi que cellulaire et subcellulaire de ce composé et des rétinoïdes.

15. Utilisation de l'acide 4-(5,6,7,8-tétrahydro-5,5,8,8-tétraméthyl-2-anthracényl) benzoïque tritié comme marqueur radioactif pour étudier le métabolisme de ce composé in vivo et dans les cellules et extraits cellulaires.

## Claims

1. Tritium-labelled 4-(5,6,7,8-tetrahydro-5,5,8,8-tetramethyl-2-anthracenyl)benzoic acid.

2. Tritiated 4-(5,6,7,8-tetrahydro-5,5,8,8-tetramethyl-2-anthracenyl)benzoic acid as defined in the preceding claim, characterised in that it has a specific activity of at least 1875 GBq/mmole.

3. Process for the preparation of a tritiated product as defined in either of the preceding claims, characterised in that:
- either a polyhalogenated alkyl ester of 4-(5,6,7,8-tetrahydro-5,5,8,8-tetramethyl-2-anthracenyl)benzoic acid is prepared and is subjected to the action of a tritiated reducing agent or of tritium in the presence of a catalyst and then to a saponification;
- or the alkyl ester of 4-(5,6,7,8-tetrahydro-5,5,8-8-tetramethyl-2-anthracenyl)benzoic acid is subjected to an isotope exchange reaction with tritiated water in the presence of platinum and of acetic acid at a temperature of 60°C to 150°C.

4. Process according to Claim 3, characterised in that the reducing agent is tritium.

5. Process according to either of Claims 3 and 4, characterised in that the halogenated product is obtained by the action of a halogenating agent on an alkyl ester of 4-(5,6,7,8-tetrahydro-5,5,8,8-tetramethyl-2-anthracenyl)benzoic acid.

6. Process according to any one of Claims 3 to 5, characterised in that the halogenating agent is a brominating agent.

7. Process according to any one of Claims 3 to 5, characterised in that the alkyl ester employed is the ethyl ester.

8. Use of tritiated 4-(5,6,7,8-tetrahydro-5,5,8,8-tetramethyl-2-anthracenyl)benzoic acid as a radioactive tracer for detecting, determining or labelling nuclear retinoic acid receptors, endogenic or obtained by transfaction methods.

9. Use of tritiated 4-(5,6,7,8-tetrahydro-5,5,8,8-tetramethyl-2-anthracenyl)benzoic acid as a radioactive tracer for measuring the affinity of the retinoids for the RARs (competition experiments) and obtaining an estimate of their biological activity.

10. Use of tritiated 4-(5,6,7,8-tetrahydro-5,5,8,8-tetramethyl-2-anthracenyl)benzoic acid as a radioactive tracer for detecting, determining or labelling the CRABP.

11. Use of tritiated 4-(5,6,7,8-tetrahydro-5,5,8,8-tetramethyl-2-anthracenyl)benzoic acid as a radioactive tracer for measuring the affinity of the retinoids for the CRABP.

12. Use of tritiated 4-(5,6,7,8-tetrahydro-5,5,8,8-tetramethyl-2-anthracenyl)benzoic acid as a radioactive tracer for determining a known retinoid by competition for binding onto the CRABP.

13. Use of tritiated 4-(5,6,7,8-tetrahydro-5,5,8,8-tetramethyl-2-anthracenyl)benzoic acid as a radioactive tracer for the characterisation of antibodies directed against this compound and the combined use of the compound and of the antibodies to perform a radioimmunoassay.

14. Use of tritiated 4-(5,6,7,8-tetrahydro-5,5,8,8-tetramethyl-2-anthracenyl)benzoic acid as a radioactive tracer for studying the in-vivo and cellular and subcellular distribution of this compound and of the retinoids.

15. Use of tritiated 4-(5,6,7,8-tetrahydro-5,5,8,8-tetramethyl-2-anthracenyl)benzoic acid as a radioactive tracer for studying the metabolism of this compound in vivo and in cells and cell extracts.

## Patentansprüche

1. Tritiummarkierte 4-(5,6,7,8-Tetrahydro-5,5,8,8-tetramethyl-2-anthracenyl)bezoesäure.

2. Tritiummarkierte 4-(5,6,7,8-Tetrahydro-5,5,8,8-tetramethyl-2-anthracenyl)benzoesäure gemäß dem vorhergehenden Anspruch, dadurch gekennzeichnet, daß es eine spezifische Aktivität von mindestens 1875 GBq/mMol besitzt.

3. Verfahren zur Herstellung eines tritiummarkierten Produktes gemäß einem der vorhergehenden Ansprüche, dadurch gekennzeichnet, daß man:
entweder einen mehrfach halogenierten Alkylester der
4-(5,6,7,8-Tetrahydro-5,5,8,8-tetramethyl-2-anthracenyl)-benzoesäure herstellt und auf diesen ein tritiiertes Reduktionsmittel oder Tritium in Gegenwart eines Katalysators einwirken läßt und anschließend verseift;
oder den Alkylester der 4-(5,6,7,8-Tetrahydro-5,5,8,8-tetramethyl-2-anthracenyl)-benzoesäure einer Isotopen-Austauschreaktion mit Tritiumwasser in Gegenwart von Platin und Essigsäure bei einer Temperatur von 60°C bis 150°C unterzieht.

4. Verfahren nach Anspruch 3, dadurch gekennzeichnet, daß das Reduktionsmittel Tritium ist.

5. Verfahren nach einem der Ansprüche 3 oder 4, dadurch gekennzeichnet, daß man das halogenisierte Produkt durch Einwirken eines Halogenierungsmittels auf einen Alkylester der 4-(5,6,7,8-Tetrahydro-5,5,8,8-tetramethyl-2-anthracenyl)-benzoesäure erhält.

6. Verfahren nach einem der Ansprüche 3 bis 5, dadurch gekennzeichnet, daß das Holgenierungsmittel ein Bromierungsmittel ist.

7. Verfahren nach einem der Ansprüche 3 bis 5. dadurch gekennzeichnet, daß der verwendete Alkylester der Ethylester ist.

8. Verwendung der tritiierten 4-(5,6,7,8-Tetrahydro-5,5,8,8-tetramethyl-2-anthracenyl)-benzoesäure als radioaktiver Marker zur Lokalisierung, Bestimmung oder Markierung von endogenen oder durch Transfektionsmethoden erhaltenen Kern-Rezeptoren von Retinsäure.

9. Verwendung von tritiierten 4-(5,6,7,8-Tetrahydro-5,5,8,8-tetramethyl-2-anthracenyl)-benzoesäure als radioaktiver Marker zur Bestimmung der Affinität von Retinoiden gegenüber RARs (Konkurrenzexperimente) und zur Abschätzung ihrer biologischen Aktivität.

10. Verwendung von tritiierter 4-(5,6,7,8-Tetrahydro-5,5,8,8-tetramethyl-2-anthracenyl)-benzoesäure als radioaktiver Marker zur Lokalisierung, Bestimmung oder Markierung von CRABP.

11. Verwendung von tritiierter 4-(5,6,7,8-Tetrahydro-5,5,8,8-tetramethyl-2-anthracenyl)-benzoesäure als radioaktiver Marker zur Bestimmung der Affinität von Retinoiden gegenüber CRABP.

12. Verwendung von tritiierter 4-(5,6,7,8-Tetrahydro-5,5,8,8-tetramethyl-2-anthracenyl)-benzoesäure als radioaktiver Marker zur Bestimmung eines bekannten Retinoids durch Kompetition bei der Fixierung auf CRABP.

13. Verwendung von tritiierter 4-(5,6,7,8-Tetrahydro-5,5,8,8-tetramethyl-2-anthracenyl)-benzoesäure als radioaktiver Marker zur Charakterisierung von Antikörpern gegen diese Verbindung und zur gemeinsamen Verwendung dieser Verbindung mit Antikörpern zur Durchführung einer Radioimmunobestimmung.

14. Verwendung von tritiierter 4-(5,6,7,8-Tetrahydro-5,5,8,8-tetramethyl-2-anthracenyl)-benzoesäure als radioaktiver Marker zur Untersuchung der in vivo und der zellulären und subzellulären Verteilung dieser Verbindung sowie von Retinoiden.

15. Verwendung von tritiierter 4-(5,6,7,8-Tetrahydro-5,5,8,8-tetramethyl-2-anthracenyl)-benzoesäure als radioaktiver Marker zur Untersuchung des Metabolismus dieser Verbindung in vivo, in Zellen und Zellextrakten.
